(19) Europäisches Patentamt
European Patent Office
Office européen des brevets

(11) **EP 4 653 836 A1**

(12) **EUROPEAN PATENT APPLICATION**

(43) Date of publication:
**26.11.2025 Bulletin 2025/48**

(21) Application number: 25171503.3

(22) Date of filing: **18.04.2025**

(51) International Patent Classification (IPC):
***G01M 3/16*** (2006.01)     ***G01M 3/20*** (2006.01)
***G06Q 10/20*** (2023.01)     ***G01N 33/00*** (2006.01)

(52) Cooperative Patent Classification (CPC):
**G01M 3/16; G01M 3/20; G01N 33/0075;
G06Q 10/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **22.05.2024 US 202418671279**

(71) Applicant: **Honeywell International Inc.
Charlotte, NC 28202 (US)**

(72) Inventor: **KASTELEIN, Bas
Charlotte, 28202 (US)**

(74) Representative: **Houghton, Mark Phillip
Patent Outsourcing Limited
1 King Street
Bakewell, Derbyshire DE45 1DZ (GB)**

(54) **SYSTEMS FOR PROBABILITY BASED FUGITIVE GAS LEAK DETECTION**

(57) An illustrative system for probability based fugitive gas leak detection comprises an equipment group including: a potential fugitive gas source; and a plurality of gas sensors positioned at respective locations in proximity to the potential fugitive gas source, wherein each of the plurality of gas sensors is configured to detect gas concentrations over a time period, and a supervisor communicatively coupled to the equipment group, the supervisor being configured to: receive the detected gas concentrations; determine a probability matrix based at least on the coordinates of the gas sensors and coordinates of the potential fugitive gas source; and identify the potential fugitive gas source as an actual fugitive gas source based on the probability matrix and the detected gas concentrations.

FIG. 1

**EP 4 653 836 A1**

**Description**

TECHNICAL FIELD

The present disclosure relates generally to systems and methods relating to atmospheric monitoring for fugitive gas leaks, and more particularly to systems and method for probability based fugitive gas leak detection.

BACKGROUND

In many jurisdictions, stringent regulations and laws govern industrial gas emissions, reflecting growing concerns over environmental impact and public health. These regulations encompass a wide range of industrial gases and/or pollutants, with particular emphasis on industrial greenhouse gases like methane, known for their potent contribution to climate change. Industrial activities, including manufacturing, energy production, and waste disposal, are significant sources of these emissions. Beyond exacerbating global warming, these emissions can degrade air quality, pose health risks to nearby communities, and harm ecosystems. Moreover, the release of certain gases, such as volatile organic compounds (VOCs), can contribute to the formation of ground-level ozone and smog, further compromising air quality and public health.

While some emissions occur through controlled processes, such as combustion in industrial furnaces or power plants, others are released unintentionally or through leaks in equipment and infrastructure. These fugitive gas emissions, often unnoticed or unregulated, can represent a significant challenge for regulatory compliance and environmental management. Inadvertent releases of large quantities of industrial gases, particularly methane, underscore the economic and environmental costs associated with fugitive emissions.

SUMMARY

The present disclosure relates generally to methods and systems for detecting and quantifying fugitive emissions, more particularly to detecting and quantifying fugitive gas emissions via a gas sensor (e.g., point sensor) network and probability based methodology in the absence (without) of real-time wind data (e.g., in the absence of an anemometer).

An example may be found in a system for probability based fugitive gas leak detection. The system comprising: an equipment group including: a plurality of potential fugitive gas sources; and a plurality of gas sensors positioned at respective locations in proximity to the plurality of potential fugitive gas sources, wherein each of the plurality of gas sensors is configured to detect gas concentrations over a time period; and a supervisor communicatively coupled to the equipment group, the supervisor being configured to: receive the detected gas concentrations; determine a probability matrix based at least on coordinates of the plurality of gas sensors and coordinates of the plurality of potential fugitive gas sources; and identify, from the plurality of potential fugitive gas sources, a potential fugitive gas source as an actual fugitive gas source based on the probability matrix and the detected gas concentrations.

Another example may be found in a system for probability based fugitive gas leak detection. The system comprising an equipment group including: a plurality of potential fugitive gas sources; and a plurality of gas sensors positioned at respective locations in proximity to the plurality of potential fugitive gas sources, wherein each of the gas sensors is configured to detect gas concentrations over a time period. The system comprising a gateway communicatively coupled to the plurality of gas sensors, the gateway configured to receive time-series data (e.g., including one or more gas events) indicative of the detected gas concentrations. The system comprising a supervisor communicatively coupled via the gateway to the equipment group, the supervisor being configured to: receive, via the gateway, the time-series data indicative of the detected gas concentrations; determine a probability matrix based at least on coordinates of the plurality of gas sensors and coordinates of the potential fugitive gas source, wherein probability matrix includes probability values associated with each of the plurality of potential fugitive gas sources and each of the plurality of gas sensors; and based on the probability values in the probability matrix and the detected gas concentrations, identify at least one potential fugitive gas source of the plurality of potential fugitive gas sources as an actual fugitive gas source, and wherein the system is anemometer-free.

Another example may be found in a method for A method for probability based fugitive gas leak detection in an environment including a plurality of equipment groups, each of the plurality of equipment groups including a plurality of gas sensors and at least one potential fugitive gas source. The method comprising: receiving, via a network, time-series data over a rolling time window, wherein the time-series data is indicative of gas concentrations detected by the plurality of gas sensors in the plurality of equipment groups during the rolling time window; determining a probability matrix for each equipment group of the plurality of equipment groups, wherein the probability matrix is determined based at least on coordinates of the gas sensors and coordinates of the potential fugitive gas source, and wherein the probability matrix includes probability values that correspond to combinations of an individual gas sensor and an individual potential fugitive gas source and indicate a probability that a gas concentration detected by the individual gas sensor originated from the individual potential fugitive gas source; based at least on the gas concentrations detected by the plurality of gas sensors and the probability values the probability matrix for an equipment group of the plurality of equipment group, identifying a potential fugitive gas source as a fugitive gas source; quantifying a volume of fugitive gas emitted by the actual fugitive gas source during the rolling time window; and remediating the actual fugitive gas source.

The preceding summary is provided to facilitate an understanding of some of the innovative features unique to the present disclosure and is not intended to be a full description. A full appreciation of the disclosure can be gained by taking the entire specification, claims, figures, and abstract as a whole.

## BRIEF DESCRIPTION OF THE FIGURES

The disclosure may be more completely understood in consideration of the following description of various examples in connection with the accompanying drawings, in which:

FIG. 1 is a schematic block diagram of an illustrative system for probability based fugitive gas leak detection;
FIG. 2 is a schematic block diagram of another illustrative system including equipment groups for probability based fugitive gas leak detection;
FIG. 3 is a flow diagram showing an illustrative method for probability based fugitive gas leak detection; and
FIG. 4 is a flow diagram showing another illustrative method for probability based fugitive gas leak detection.

While the disclosure is amenable to various modifications and alternative forms, specifics thereof have been shown by way of example in the drawings and will be described in detail. It should be understood, however, that the intention is not to limit the disclosure to the particular examples described. On the contrary, the intention is to cover all modifications, equivalents, and alternatives falling within the spirit and scope of the disclosure.

## DESCRIPTION

The following description should be read with reference to the drawings, in which like elements in different drawings are numbered in like fashion. The drawings, which are not necessarily to scale, depict examples that are not intended to limit the scope of the disclosure. Although examples are illustrated for the various elements, those skilled in the art will recognize that many of the examples provided have suitable alternatives that may be utilized.

All numbers are herein assumed to be modified by the term "about", unless the content clearly dictates otherwise. The recitation of numerical ranged by endpoints includes all numbers subsumed within that range (e.g., 1 to 5 includes, 1, 1.5, 2, 2.75, 3, 3.8, 4, and 5).

As used in this specification and the appended claims, the singular forms "a", "an", and "the" include the plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is contemplated that the feature, structure, or characteristic may be applied to other embodiments whether or not explicitly described unless clearly stated to the contrary.

It may be desirable to detect and quantity fugitive gas emissions. For instance, various plants such as chemical manufacturing plants, petroleum refineries and other industrial facilities may inventory potential fugitive gas sources. Examples of potential fugitive gas sources include all industrial equipment such as valves, pumps, flanges, burners, etc., that could potentially be a source of fugitive gas emissions. The plants may perform routine manual testing (e.g., via a hand-held gas detector) of the potential fugitive gas sources. However, such approaches may be time-consuming, prone to error, costly, and/or may not detect intermittent fugitive gas leaks, at least due to the periodic nature of the manual inspections. For instance, such approaches may conduct the manual testing infrequently and therefore may be prone to not detecting intermittent fugitive gas leaks. Yet, it has been determined that intermittent leaks may be a significant factor in overall fugitive gas emissions.

Other approaches may employ a continuous emission monitoring system to detect potential fugitive gas emissions (e.g., intermittent fugitive leaks). Continuous Emission Monitoring systems have been developed that are able to continuously measure gas (e.g., methane) emissions instead of periodically, e.g. through camera's, laser systems or point sensor networks. For instance, gas sensor networks (e.g., point sensor networks located on a fence) may measure a concentration of methane with gas sensors that are positioned adjacent to a potential fugitive gas source. For example, the gas sensors may yield measured time-series gas (e.g., methane) concentration data. However, such approaches may rely on the time-series data being correlated with dynamic (real-time) wind direction and wind speed data, as obtained from a local anemometer or weather station to permit detection and/or quantification of a fugitive gas emission. Yet, due at least to the reliance on the dynamic (real-time) wind direction and speed data such approaches may be costly (e.g., an anemometer that is certified for use in an industrial environment and/or hazardous area may be costly), require the presence and maintenance of various hardware (e.g., an anemometer and cables/communication hardware) and/or may be prone to failure. For instance, in an industrial plant with dense equipment the wind direction and/or wind speed at a potential fugitive gas source (e.g., equipment unit under monitoring) can significantly deviate from a wind speed and wind direction as measured by a distant anemometer. Thus, the fugitive leak detection and/or quantification predicated on the inaccurate wind data may have various issues (e.g., may not detect a fugitive gas leak, may attribute a fugitive gas leak to the incorrect potential fugitive gas emission source, may not accurately quantify a fugitive gas leak, etc.).

Moreover, some approaches may employ gas sensors that are not hazardous area certified. As a result, the gas sensors may be positioned a large distance away from a potential fugitive gas emission source such as being positioned on a fence line. As such, these approaches may be inaccurate (e.g., in terms of detecting and/or quantifying a fugitive gas emission), at least due to the relatively large distance between the gas sensors and the potential fugitive gas emission sources.

As such, the present disclosure provides system and methods to accurately detect and quantify fugitive gas emissions in the absence of an anemometer (e.g., in the absence of wind speed and wind direction data). The present disclosure employs a probability based approach associated with a relatively small quantity of sensors (e.g., a relatively small quantity of sensors associated with given potential fugitive gas source in an equipment group positioned adjacent to (in the direct vicinity of) the equipment group. For instance, the present disclosure may employ time-series data and probability matrices associated with a plurality of hazardous area gas sensors that are located a threshold distance (e.g., about 1 meter to about 10 meters) from a potential fugitive gas emission source thereby reducing the need for a local anemometer and yet permitting the detection, localization and quantification of fugitive gas emissions (e.g., continuous and intermittent fugitive gas emissions), as detailed herein.

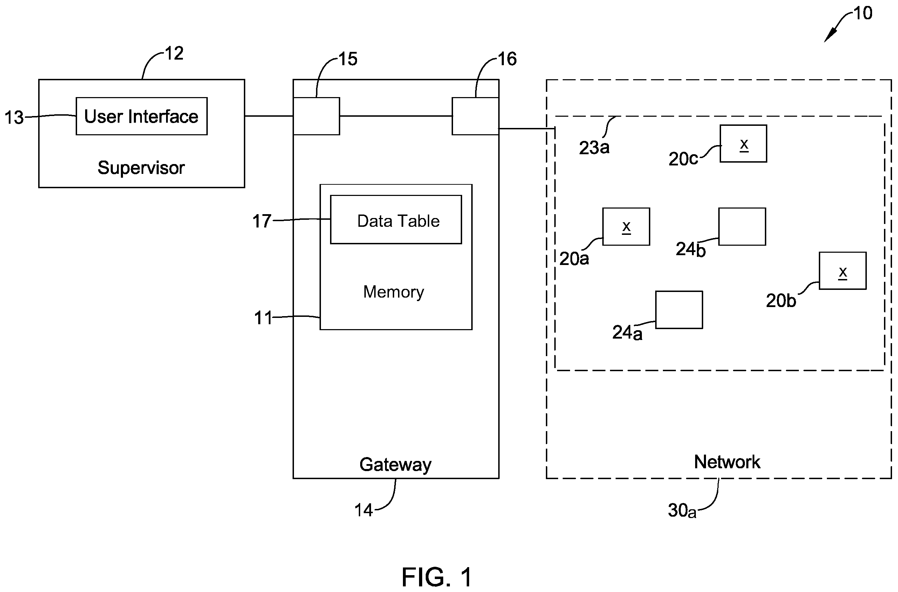

Figure 1 is a schematic block diagram showing an illustrative system 10. The system 10 may be deployed in an industrial plant that is suitable for oil & gas, pharmaceutical, chemical, food & beverage, and/or other applications. The illustrative system 10 includes a supervisor 12, a headend device such as a gateway 14 (e.g., a gateway hub), and a plurality of gas sensors 20a, 20b, 20c (e.g., as designated with a boxes including an "X" therein).

The system 10 can include at least one equipment group 23. As illustrated in FIG. 1, the equipment group 23 can include a plurality of potential fugitive gas sources including a first fugitive gas source 24a and a second fugitive gas source 24b. As illustrated in FIG. 1, the equipment group 23 can include the plurality of gas sensors 20a, 20b, 20c positioned at respective locations in proximity (e.g., less than 20 meters, less than 15 meters, less than 10 meters, etc.) to the potential fugitive gas sources. Identifying information (e.g., a name and/or status of the equipment group and/or a name and/or status of equipment within the equipment group) can be stored in the supervisor 12.

The supervisor 12 may be manifested as an application executing on a computer such as a desktop/laptop computer, computer server and/or a smartphone, among other possibilities. The supervisor 12 may include a user interface 13. In some cases, the user interface 13 may be a display for displaying information. In some cases, the user interface 13 may include a data entry device such as a keyboard, mouse, trackball or electronic writing surface. In some cases, the user interface 13 may include a touch screen that functions as a display as well as providing data entry functionality.

The supervisor 12 may be used to receive emissions data from plant operations. For instance, the supervisor 12 can be configured to receive time-series data that is representative of detected gas concentrations. The supervisor can received the time-series data directly from one or more gas sensors in an environment in which a plant is located and/or can received the time-series data indirect (e.g., access the time-series data from a storage location such as cloud storage). The supervisor 12 may include centralized software that coordinates and simplifies workflow by orchestrating data from sensing sources, while calculating and aggregating near real-time emissions information to deliver transparency and status across the plant or other type of deployment location. For instance, the supervisor can collect and store real-time data (e.g., real-time gas data) detected by various sensors such as the gas sensor 20a, 20b, 20c. The supervisor 12 can also store a list of a plurality of potential fugitive gas sources such as a potential fugitive gas sources 24a, 24b, illustrated in FIG. 1. While an individual external controller in the form of individual supervisor 12, is shown, it will be appreciated that this is merely illustrative, as the system 10 may include any number of external controllers (e.g., supervisors).

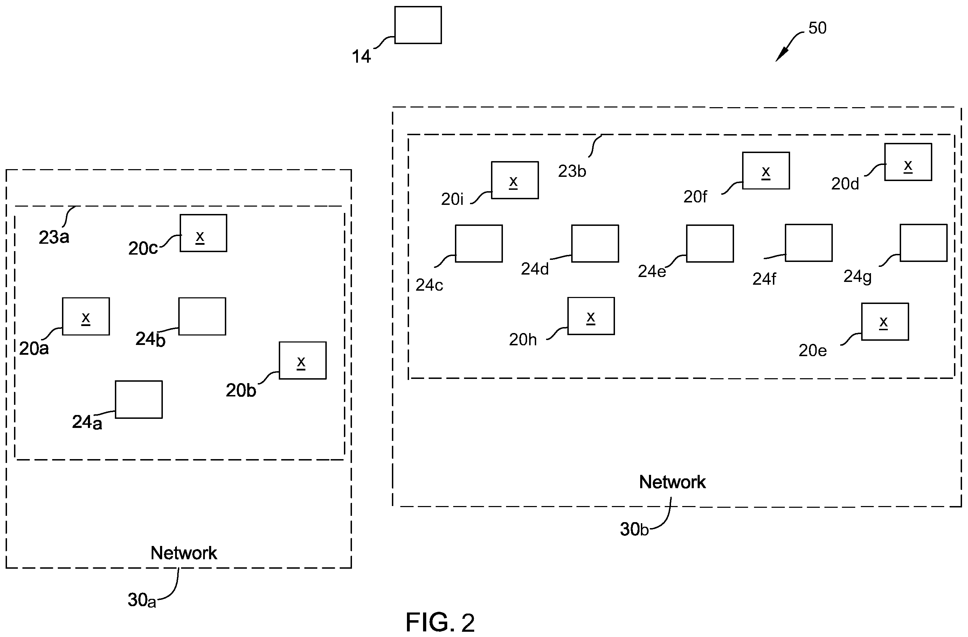

In some embodiments, the supervisor 12 (e.g., an enterprise emission manager) can be configured to cause the user interface 13 to display a device identifier for each of one or more of gas sensors 20a, 20b, 20c, and/or a device identifier for each of the plurality of potential fugitive gas sources. In some embodiments, the supervisor 12 can be configured to cause the user interface 13 to display a representation of a physical layout (e.g., a bird's eye view) of the gas sensors and the plurality of potential fugitive gas sources, as detailed in FIG. 2. For instance, in some embodiments, the supervisor 12 can be configured to provide a real-time representation of each a plurality of equipment groups such as the equipment group 18 including sensors 20a, 20b, 20c and the plurality of potential fugitive gas sources. In such embodiments, the supervisor 12 can be configured to display a real-time representation of any detected fugitive emissions, as detailed herein. For instance, the supervisor 12 can be configured to display via the user interface 13 a representation and/or identifying gas events associated with one or more potential fugitive gas sources determined to be an actual fugitive gas source (e.g., that is experiencing a real-time fugitive gas event).

The gateway 14 can be coupled via a first port 15 to an external controller such as the supervisor 12. The gateway 14 can communicate via the first port 15 with the external controller such as the supervisor 12. In some embodiments, the gateway 14 can be a LoRa gateway. The gateway 14 can be communicative coupled to the supervisor 12 and the gas sensors 20a, 20b, 20c in a wired or wireless manner (via a cellular, LoRaWAN, Wi-Fi, and/or Bluetooth, etc.).

The gateway 14 can be coupled via a second port 16 to the gas sensors 20a, 20b, 20c over a wired and/or wireless network. While an individual gateway 14 is shown, it will be appreciated that this is merely illustrative, as the system 10 may include any number of gateways. For instance, the gateway 14 can include a memory 11 to store detected gas concentrations detected by the gas sensors 20a, 20b, 20c. For instance, the gateway 14 can store that detected gas concentrations as time-series data in a data table 17 in the memory 11 of the gateway 14. The memory 11 can be a volatile memory, a non-volatile memory, or a combination thereof.

The plurality of gas sensors 20a, 20b, 20c can be configured (e.g., with spectrometers) to detect a respective concentrations of a gas over a time period. The gas sensors herein are configured to detect hydrogen, hydrogen sulfide, carbon dioxide, methane, carbon monoxide, or any combination thereof. For instance, the gas sensors herein can be configured to detect various volatile organic compounds such as methane. An example of a suitable gas sensor configured to detect at least methane is the Honeywell Versatilis™ Signal Scout™ which is available from HONEYWELL INTERNATIONAL INC.

In some embodiments, at least some of the gas sensors are a hazardous area certified. For instance, each of the gas sensors can be hazardous area certified. As used herein, being hazardous area certified refers to having one or more hazardous certification selected from a group consisting of International Electrotechnical Commission System for Certification to Standards Relating to Equipment for Use in Explosive Atmospheres (IECEx System) certification, an ATEX, Intrinsic Safety & Hazardous Area Information certification (e.g., Exi-a) certification, and a C1D1 certification. For instance, the gas sensors may have each of an (IECEx System) certification, an ATEX, Intrinsic Safety & Hazardous Area Information certification (e.g., Exi-a) certification, and a C1D1 certification.

Employing gas sensors that are hazardous area certified can promote aspects herein. For instance, the gas sensors can be located external to and a distance away from the potential fugitive gas source, yet can be located relatively close to the potential fugitive gas source (e.g., unlike non-hazardous area certified gas sensors that must be positioned further away from the potential fugitive gas source). In some embodiments, each of the gas sensor can be located a distance that is in a range from about 1 meter to about 20 meters from the potential fugitive gas source. All individual values and sub-ranges from about 1 meter to about 20 meters are included. For instance, the gas sensors can be located a distance that is in a range from about 1 to about 20 meters, about 1 meter to about 15 meters, about 1 to about 10 meters, about 1 to about 5 meters, about 5 to about 10 meters, or from about 5 to about 15 meters from the potential fugitive gas source. Thus, in contrast to other approaches that employ large quantities of gas sensors (e.g., gas sensors positioned at a large distance on a fence line), the approaches herein employ a relatively small quantity of gas sensors.

For instance, in some embodiments a ratio of gas sensors to a potential fugitive gas source can be 1:1, 2:1, 3:1, 4:1 or 5:1. For example, in some embodiments, a ratio of gas sensors to a potential fugitive gas source can be 4:1. In some embodiments a ratio of gas sensors to a potential fugitive gas source can be 1:1. In some embodiments a ratio of gas sensors to a potential fugitive gas source can be 2:1. In some embodiments a ratio of gas sensors to a potential fugitive gas source can be 3:1. In some embodiments a ratio of gas sensors to a potential fugitive gas source can be 4:1. In some embodiments a ratio of gas sensors to a potential fugitive gas source can be 5:1. Other ratios are possible.

In some embodiments, the gas sensors can be located at fixed locations (e.g., that is a fixed distance away from the potential fugitive gas source). Having the gas sensors be located at fixed locations (e.g., at least with respect to the potential fugitive gas source in the same equipment group) can promote aspects herein such as promoting the timely and accurate detection and quantification of fugitive gas leaks. For instance, a respective fixed location (e.g., respective fixed GPS coordinates) of each of the gas sensors and each of the potential fugitive gas sources can be stored, for instance, in the supervisor 12. Thus, the respective fixed locations can be employed to facilitate aspects herein such as detection and quantification of a fugitive gas leak, as detailed herein.

In some embodiments each of the gas sensors herein such as the gas sensors 20a, 20b, 20c can be positioned substantially the same distance away from the potential fugitive gas source 24, as illustrated in FIG. 1. Having each of the gas sensors can be positioned substantially the distance away from a potential fugitive gas source can promote aspects herein such a promoting timely and accurate detection and quantification of fugitive gas leaks from the potential fugitive gas source. However, in some embodiments, one or more of the gas sensors can be positioned a different respective distance away from the potential fugitive gas source. In such embodiments, the supervisor 12 can account for the differences in distances (e.g., attribute more weight to a closer gas sensor) and thus can still permit timely and accurate detection and quantification of fugitive gas leaks from the potential fugitive gas source.

In some embodiments, each of the gas sensors in an individual equipment group can be located a different location. For instance, a first gas sensor 20a can be located on a first side of a potential fugitive gas source such as the potential fugitive gas source 24a, a second gas sensor 20b can be located on a second side of the potential fugitive gas source 24a, a third gas sensor 20c can be located on a third side of the potential fugitive gas source 24a. Having each of the gas sensors in the equipment group be based at different positions can promote aspects herein (e.g., promote detection and quantification of a fugitive gas leak under varying wind conditions).

In some embodiments, each of the gas sensors in an individual equipment group can be configured to detect a respective concentrations of a gas at substantially the same time or at exactly the same time. Having each of the gas sensors in an individual equipment group be configured to detect a respective gas concentrations at substantially the same time (or at exactly the same time) can promote aspects herein. For instance, each of the gas sensors can be configured to detect respective gas concentrations periodic gas measurements at the same time and interval. Thus, in some instances the respective gas concentrations are real-time measurements that are detected at substantially the same time by each of the gas sensor in a respective equipment group. For instance, each of the gas sensors in a respective equipment group can be configured to detect respective gas concentrations periodically (e.g., substantially continuously) such as every second, every 5 seconds, every 10 seconds, or every 20 seconds, among other possibilities. However, in some instances the gas sensors may be configured to detect respective gas concentrations continuously over a time period.

While FIG. 1 illustrates the presence of three gas sensors 20a, 20b, 20c this is merely illustrative, as the equipment group 23 may each include any number gas sensors. The devices within the network 30a communicate (in a wired and/or wireless manner) with the other devices within the network 30a as shown. One or more of the gas sensors in an equipment group can be configured to communicate with the gateway 14. Each of the gas sensors may independently be any of a variety of different gas sensors and/or other device such as other IoT devices.

The system 10 is anemometer-free (i.e., does not include an anemometer). That is, the system 10 permits timely and accurate detection and quantification of a fugitive gas leak in the absence of an anemometer/dynamic weather data (such as wind speed and wind direction), as detailed herein.

FIG. 2 is schematic representation of another illustrative system 50 for probability based fugitive gas leak detection. The system 50 includes a gateway 14 and a plurality of equipment groups 23a and 23b. While FIG. 2, illustrates the presence of an individual gateway 14 and two equipment groups, 23a and 23b, the system 50 can include any quantity of equipment groups and/or gateways. The gateway 14 is analogous or similar to the gateway 14 described with respect to FIG. 1. For instance, the gateway 14 may be communicatively coupled (e.g., in a wireless or wired manner) to each of the equipment groups 23a, 23b. The system 50 can include additional elements such as a supervisor, etc.

As illustrated in FIG. 2, each of the equipment groups 23a, 23b include a plurality of gas sensors. For instance, the first equipment group 23a includes a plurality of gas sensors 20a, 20b, 20c located therein, and the second equipment group 23b includes a plurality of gas sensors 20d, 20e, 20f, 20h, 20h located therein, as illustrated in FIG. 2. As illustrated in FIG. 2, in some embodiments respective equipment groups can have different respective quantities of gas sensors therein. Similarly, in some embodiments respective equipment groups can have different respective quantities of potential fugitive gas sources therein. However, in some embodiments different equipment groups can include an equal quantity of potential fugitive gas sources and/or an equal quantity of gas sensors.

Some or all of the gas sensors in a given equipment group can facilitate the detection and quantification of fugitive gas leaks, as detailed herein. For instance, continuing with the discussion of FIG. 2, each of the gas sensors 20a, 20b, 20c in the first equipment group 23a can detect gas concentrations and thereby permit the detection and quantification of fugitive gas leaks associated with the potential fugitive gas source 24a, as detailed herein. The detection and quantification of fugitive gas leaks can occur based on at least the detected gas concentrations, coordinates of the gas sensors (those that detected the gas concentrations), coordinates of the potential fugitive gas source(s), and/or a distance between the fugitive gas sensors and the potential fugitive gas sources, etc. For instance, gas sensors that are closer to a potential fugitive gas source and/or that detects a relatively high gas concentration (e.g., as compared to gas concentrations detected by other gas sensors) may indicate that the potential fugitive gas source has a higher probability of being an actual fugitive gas source (e.g., that is experiencing a real-time fugitive gas leak). Conversely, a gas sensor that is less proximate to the potential fugitive gas source and/or that detects a relatively low gas concentration may indicate that the potential fugitive gas source has a lower probability of being the actual fugitive gas source, as detailed herein.

In some embodiments, a distance threshold can be employed. In such embodiments, gas concentrations detected by gas sensors that are located a distance that is greater than the distance threshold can be omitted. Employing a distance threshold can reduce computation time, reduce false positives and/or otherwise promote aspects herein. For instance, in some embodiments a distance threshold can be associated with each potential fugitive gas source. In such embodiments the distance threshold can ensure that a subset but not all gas sensor concentrations are utilized when determining the occurrence of a fugitive gas event. For instance, that distance threshold associated with a given potential fugitive gas source can be configured to include only gas sensors that are within the same equipment group as the potential fugitive gas source, among other possibilities. Stated differently, only potential fugitive gas sources that are located a distance that is less than the distance threshold may be considered when detecting an occurrence of a fugitive gas event.

That is, potential fugitive gas sources that are located greater than the threshold distance from a given gas sensor will may not be determined to be a detected source of a fugitive gas event. Without wishing to be bound by theory or a particular implementation, a rationale for applying a distance threshold is that detection probability reduces with distance, as the concentration dilutes, and also because of air turbulence, causing gas molecules that are blown by the wind from the gas source towards the gas sensor, will deviate their track and will not reach the gas sensor. This limited detection range may also help to confine leak detections to within an individual equipment group.

Table 1 illustrates an example of a probability matrix associated with the equipment group 23b. As detailed herein, the probability values in the probability matrix can facilitate aspects herein such as the timely and accurate detection of a fugitive gas leak (e.g., identification of a potential fugitive gas source as an actual gas source) in the absence of real-time wind data. For instance, as illustrated in Table 1 the probability matrix can include each of the potential fugitive gas sources (e.g., 24c, 24d, 24e, 24f, and 24e) and each of the gas sensors (20d, 20e, 20f, 20h, and 20i) in the equipment group (e.g., 23b). Some or all combinations of the individual gas sensors and the individual potential fugitive gas sources can have a corresponding probability value. As detailed herein, the probability value can be based at least on a distance between an individual gas sensors and an individual gas sensor. For instance, values the represent a distance (e.g., an actual distance or a normalized distance) can be employed, among other possibilities. As mentioned, a higher probability value (e.g., 1.0) represents a higher probability that the individual potential fugitive gas source is an actual fugitive gas source than a lower probability value (e.g., 0.2). For example, as denoted in Table 1 the gas sensors 20d (having a probability value of 1.0) and the gas sensor 20e (having a probability value of 0.8)

Table 1

| | | Potential fugitive gas sources | | | | |
|---|---|---|---|---|---|---|
| | | 24g | 24f | 24e | 24d | 24c |
| Gas Sensors | 20f | 0.2 | 0.8 | 1.0 | 0.2 | - |
| | 20e | 0.8 | 0.8 | - | - | - |
| | 20h | - | - | 0.3 | 0.8 | 1.0 |
| | 20i | - | - | 0.1 | 0.8 | 0.8 |
| | 20d | 1.0 | 0.3 | - | - | - |

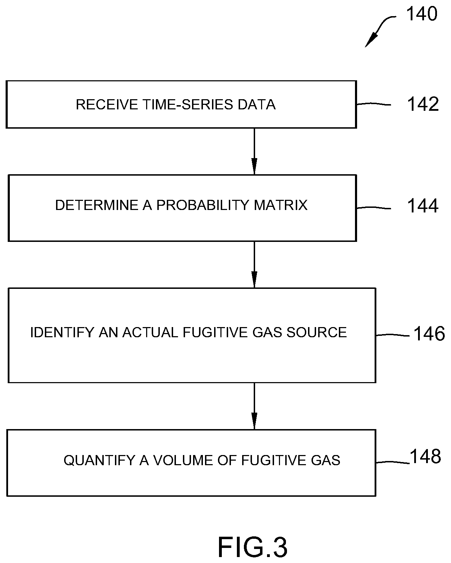

FIG. 3 is a flow diagram showing an illustrative method 140 for probability based fugitive gas leak detection. The method can be performed with the systems herein.

The method 140 can be performed periodically or continuously. That is, in some embodiments the probability based fugitive gas leak detection can occur periodically (e.g., every few seconds, every few minutes, etc.), as detailed herein. For instance, the gas sensors can detect gas concentrations every few seconds, in some embodiments. However, in some embodiments the gas sensors can detect gas concentrations continuously. Responsive to detection of one or more gas concentrations, the gas sensors can transmit time-series data (e.g., representative of one or more gas events) indicative of the one or more gas concentrations to a gateway and the gateway can subsequently transmit that information indicative of the one or more gas concentrations to a supervisor, as detailed herein.

At block 142, the method 140 can include receiving, via a network (e.g., network 30a and/or network 30b), time-series data indicative of gas concentrations detected by the plurality of gas sensors in the plurality of equipment groups over a time period. For instance, the time-series data that is indicative of one or more gas concentrations detected by each of the gas sensors in an equipment group can be received by the supervisor. In some embodiments, the method 140 can include receiving, via a communication network, time-series data indicative of gas concentrations of one or more gas events detected by the plurality of gas sensors during a rolling time window.

At block 144, the method 140 can include determining a probability matrix for each equipment group of the plurality of equipment groups. The probability matrix can be determined prior to or subsequent to receipt of the receipt of the time-series data indicative of gas concentrations detected by the plurality of gas sensors in the plurality of equipment groups over a time period. For instance, in some embodiments, the probability matrix for each of the equipment groups can be determined prior to detection of the gas concentrations. In this way, the probability matrices for each of the equipment groups can be initially determined and can be stored (e.g., have values that remain unchanged) to permit the subsequent timely and accurate determination and quantification of any fugitive gas leaks based on the probability matrices and the detected gas concentrations, as detailed herein. For instance, the probability matrices can be stored in the supervisor and/or otherwise stored (e.g., in a cloud server) and be retrievable by the supervisor.

As mentioned, the probability matrix can be determined based at least on the coordinates of the gas sensors and coordinates of the potential fugitive gas sources. Stated differently, the probability matrix can be determined based at least on actual distances between (e.g., a quantity of meters therebetween) the gas sensors and the potential fugitive gas sources. For instance, the probability matrix can include values that represent or are a function of a distance between the coordinates of the gas sensors and the coordinates of the potential fugitive gas sources. For example, a probability value in the probability matrix can indicate an actual distance between an individual gas sensor in an equipment and an individual potential fugitive gas source. In such instances, the distance can be determined based on a difference between the GPS coordinates of the individual gas sensor and the GPS coordinates of the individual potential fugitive gas sources, among other possibilities.

For example, an actual distance between an individual gas sensor and an individual potential fugitive gas source that is relatively small can have a higher probability value, thus indicating a higher probability that the individual potential fugitive gas source (e.g., that is relatively close to the individual gas sensor) is a fugitive gas source. Conversely, an actual distance between an individual gas sensor and an individual potential fugitive gas source that is relatively large can have a lower probability value, thus indicating a lower probability that the individual potential fugitive gas source (e.g., that is relatively far from the individual gas sensor) is a fugitive gas source.

In some embodiments, the probability values in the probability matrices can be normalized. For instance, the probability values in the probability matrices can be normalized as an inverse square root (e.g., 1/ a square of an actual distance between an individual gas sensor and an individual potential fugitive gas source. Normalizing the probability values as an inverse square root can promote aspects herein, for instance as a probability that the individual potential fugitive gas source is in fact a fugitive gas source may decrease non-linearly (e.g., quadratically) with distance.

In some examples, the method can include attributing a weight to one or more of the probability values in the probability matrices. For instance, the weight can be attributed based on an historical wind data, in some embodiments. In some embodiments, the weight can be attributed based on an average or median windspeed over a given period of time (e.g., yearly, seasonally, monthly, etc.). For example, based on historical wind data for a site, plant, or environment in which the systems herein are deployed one or more gas sensors that are "downwind" from one or more potential fugitive gas sources can have a first weight (e.g., a value that reduces probability) attributed thereto to mitigate an impact of the historical wind speed and/or historical wind direction thereon. Conversely, one or more gas sensors that are "upwind" from one or more potential fugitive gas sources can have a second weight (e.g., a value that increases probability) attributed thereto. Similarly, respective weights can be attributed based on historical wind speed. In any case, employing weights or otherwise utilizing historical wind data can promote aspects herein such as promoting accurate and timely detection and quantification of fugitive gas leaks in an absence of real-time wind data (e.g., in the absence of an anemometer). In some embodiments, the preferential (seasonal) wind direction can be weighed in in the probability matrix. For instance, each value in a cell in a probability matrix can be a product of distance detection probability and wind direction probability (e.g., a wind weight).

In some embodiments, a weight can be applied based on a mathematical model (e.g., based on an calculated Gaussian plume dispersion and/or based on machine-learning, etc. In any case, the probability matrix can include probability values (e.g., 0.1, 1.0, etc.) that correspond to combinations of an individual gas sensor and an individual potential fugitive gas source and indicate a probability that a gas concentration detected by the individual gas sensor originated from the individual potential fugitive gas source, as detailed herein.

At block 146, the method 140 can include identifying a potential fugitive gas source as an actual fugitive gas source (e.g., as experiencing an actual real-time fugitive gas leak). For instance, each of the respective probability values in the probability can be multiplied by one or more corresponding detected gas concentrations or the count of one or more gas detection events (e.g., from the same individual gas sensor) to determine a fugitive leak value. As used herein, a gas detection event refers to a detection of a non-zero gas concentration by a sensor. In some instances, the gas detection event correspond to each instance of detection of a non-zero gas concentration or each instance of detection of a non-zero gas concentration that exceeds a detection threshold.

In any case, each combination of an individual gas sensor and an individual potential fugitive gas source can have a probability-based fugitive leak value. The fugitive leak value can be based at least on: i) a distance between the individual gas sensor and the individual potential fugitive gas source; and ii) one or more gas concentrations of the gas sensors that are proximate thereto (e.g., in the same equipment group as the individual potential fugitive gas source). For instance, the fugitive leak value can be equal to a product of: i) one or more detected gas concentrations detected by an individual gas sensor; and ii) a probability value of a combination of the individual gas sensor and an individual potential fugitive gas source. For example, the fugitive leak value can be equal to a product of i) a sum, count, or average of all gas concentrations detected by an individual leak sensor during a time period; and ii) a probability value of a combination of the individual gas sensor and an individual potential fugitive gas source j. For instance, a fugitive leak value for an individual potential fugitive gas source can be determined (e.g., an individual potential fugitive gas source 24e) in an accordance with Equation 1, below.

$$FLVj = \sum_{i=0}^{ndet} Pi,j*Si \qquad \text{(Equation 1)}$$

where:

FLVj: is fugitive leak value associated with potential fugitive gas source j in an equipment group;
Pi,j: the probability value from a probability matrix associated with gas detector i and gas source j;
Si: the sum of concentrations detected over a time period by gas sensor i or is the count of gas detections over a time period by gas sensor I; and
Ndet: the number of gas sensors.

Continuing with the example probability matrix in Table 1, an illustrative calculation of a fugitive leak value for the individual potential fugitive gas source 24e may be performed as follows.

78 = (0.2 x 30 parts per million (ppm) methane detected by gas sensor 20f) + (0.8 x 50 ppm methane detected by gas sensor 20e) = (0.8 X 90 ppm methane detected by gas sensor 20d)  (Equation 2)

While the above example utilizes each of the probability values associated with the potential fugitive gas source in the probability matrix, in some instances a sub-set but not all of the probability values associated with the potential fugitive gas source in the probability matrix may be employed. Additionally, while the above example employs an individual gas concentration (e.g., 30 ppm) of an individual gas event per gas sensor over a rolling time window, in some embodiments a plurality of gas concentrations (30, 150, 75, and 100 ppm)) of a plurality gas events per gas sensor over a rolling time window may be employed.

Fugitive leak values can be determined for some or all of the potential fugitive gas sources in an equipment group in a similar manner. For instance, respective fugitive gas values can be determined for each of the potential fugitive gas sources in the equipment group. In some embodiments, a gas source with a largest fugitive leak value in an equipment group can be identified as an actual fugitive gas source. That is, in some embodiments, a potential fugitive gas source that is identified as an actual fugitive gas source has a highest fugitive leak value (e.g., a non-zero fugitive leak value that is higher than any other fugitive leak value of any other potential fugitive gas sources in the equipment group. However, in some instances a potential fugitive gas source that has a highest fugitive gas value which exceeds fugitive emission threshold can be identified as an actual fugitive gas source. That is, in some embodiments, a potential fugitive gas source having a fugitive gas value that exceeds a fugitive emission threshold, is a highest fugitive gas value (e.g., within a given equipment group), or both, can be identified as an actual fugitive gas source.

In some embodiments, the method 140 can include filtering one or more of the plurality potential fugitive gas sources prior to identifying a potential fugitive gas sources as the actual fugitive gas source. Filtering one or more of the plurality of potential fugitive gas sources refers to removing the one or more filtered gas sources from potential determination as the actual fugitive gas source. Such filtering can reduce a quantity of false positives relating to fugitive gas leaks and/or enhance the accuracy of determination of a potential fugitive gas source as an actual fugitive gas source. In some embodiments, the method can include filtering the plurality of potential fugitive gas sources using a minimum number of events filter, a minimum duration filter, a minimum event density filter, or any combination thereof. The use of additional or alternate filters is possible. As used herein, a minimum number of events refers to a minimum quantity of detected gas concentrations (non-zero detected gas concentrations) over a time period. As used herein, a minimum duration filter refers to a filter that sets a minimum duration of a detected fugitive leak (e.g., several seconds, several minutes, etc.). As used herein, a minimum event density filter refers to a filter that sets a limit (minimum) quantity of gas sensors in an equipment group that are required to detect a gas concentration (a non-zero gas concentration) prior to identifying a potential fugitive gas source as an actual fugitive gas source.

At block 148, the method 140 can include quantifying a volume of fugitive gas emitted by the actual fugitive gas source over the time period. In some embodiments, quantifying the volume of the fugitive gas emitted by the actual fugitive gas source over a time period can include determining a start and stop time of the time period and determining detected gas concentrations associated with at least two points during the time period. For example, gas concentrations can be plotted during the time period and an area (e.g., an area between and/or below the plotted gas concentrations) can be determined. The area can indicate or be equal to a quantify of fugitive gas emitted by the actual fugitive gas source.

The method 140 can include quantifying the volume of the fugitive gas emitted by the actual fugitive gas source responsive to identification of the actual fugitive gas source. That is, the approaches herein can accurately and timely identify and quantify a fugitive gas leak, unlike other approaches that may be less accurate and/or less timely (e.g., due to a reliance an a large quantity of sensors and/or reliance on real-time wind data, etc.). For example, in some instances a quantified volume of fugitive gas emitted can be determined based on a sum and/or average of each of the detected gas concentration values over the time period during which the actual fugitive gas source is experiencing a fugitive gas leak, among other possibilities.

In some embodiments, the time periods herein can occur during an on-line period (e.g., normal operational status) of a process such as a chemical, manufacturing, and/or other type of process. In some embodiments, the time periods herein can be a discrete time period having a start time and an end time. However, in some embodiments the time periods herein can be rolling-time window. In such instances, any fugitive gas leak may be detected and quantified during and/or at an end of a particular rolling time window. Thus, while various embodiments relate to the detection and quantification of fugitive gas leaks during a rolling time window while a process or plant is on-line (e.g., operating normally). For instance, the rolling time window may correspond to a time window during which one or more (e.g., all of) the plurality of potential fugitive gas sources are on-line. However, the disclosure is not so limited and in some embodiments the approaches herein may be employed during on off-line time period. In some embodiments, the fugitive gas leak can be quantified based at least in part on one or more mathematical models such as a Gaussian plume model. For instance, each gas events (e.g., a number of gas events and a respective detected gas concentration thereof) for each gas sensor associated with a fugitive gas can be summed or otherwise employed to calculate a leak rate value at the fugitive gas source. Such calculations can be based at least in part on a distance between the respective fugitive gas sensors and the fugitive gas source. For instance, such calculations can be based on a distance between the respective fugitive gas sensors and the fugitive gas source and a median seasonal wind speed value (e.g., a historical wind speed over a span of one or more months) and/or an average historical air turbulence. In such instances, a plurality of respective leak rates can be determined and an average leak rate thereof can be determined to be the quantified leak rate, among other possible mechanisms to quantify the fugitive emission.

In some embodiments, the method 140 can remediate the actual fugitive gas source. Examples of remediations include altering a process variable (e.g., a process temperature, pressure, set point, alarm point, etc.), changing or servicing component (e.g., a seal, a valve, a pipe, etc.) in a process, etc. In some examples, a type and/or a timeline of a remediation can be determined based on the quantity of fugitive gas emitted and/or a type of the fugitive gas leak (e.g., whether the fugitive gas leak is a continuous fugitive gas leak or an intermittent gas leak). That is, due at least in part to detecting intermittent gas leaks, as detailed herein, the present disclosure affords additional flexibility (e.g., in terms of a type and/or timeline) for remediating a fugitive gas leak.

For instance, in some embodiments a remediation associated with an intermittent fugitive gas leak can be scheduled as non-urgent (e.g., may occur in the future at a relevant time such as during an upcoming schedule maintenance window). Similarly, in some embodiments a remediation associated with a relatively small quantity of fugitive gas emitted by a fugitive gas leak can be scheduled as non-urgent. Conversely, in some embodiments a remediation associated with a continuous fugitive gas leak can be scheduled as urgent (e.g., may occur immediately or as relatively soon). Similarly, in some embodiments a remediation associated with a relatively large quantity of fugitive gas emitted by a fugitive gas leak can be scheduled as urgent.

In some embodiments, the method 140 can include providing a notification. For instance, the method 140 can include selecting a type of notification from a plurality of different types of notifications. For example, the supervisor can be configured to select the notification based at least on the volume of fugitive gas emitted, a fugitive gas event type, or both. The fugitive gas event type, as mentioned, can be continuous or intermittent, as described herein. In such embodiments, the selected notification can be provided via a user interface of the supervisor, or otherwise conveyed.

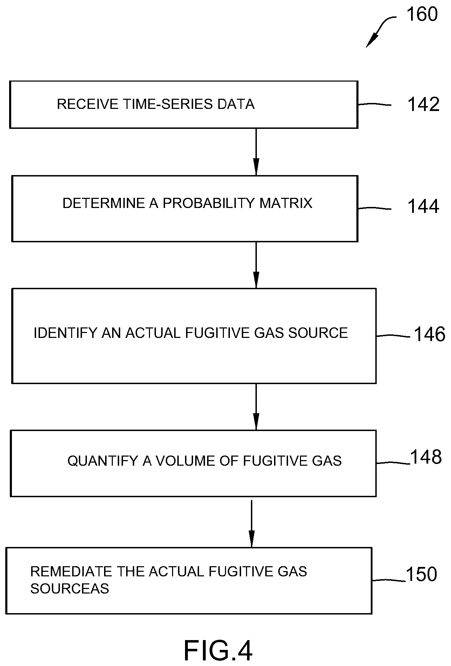

FIG. 4 is a flow diagram showing another illustrative method 160 for probability based fugitive gas leak detection. For instance, the method can be performed with the systems herein that include a supervisor, a headend device such as a gateway, and one or more equipment groups including a plurality of gas sensors and at least one potential fugitive gas source located therein. The method 160 is analogous to the method 140, but with the addition of remediating the actual fugitive gas source (e.g., remediating an actual fugitive gas leak experienced by the actual fugitive gas sources. As mentioned, a remediation can be determined based on a quantity of fugitive gas and/or a type of fugitive gas leak (e.g., whether the fugitive gas leak is continuous or intermittent). That is, the type of remediation and/or a timeline of the remediation (e.g., whether the remediation is scheduled as urgent or non-urgent) can depend upon a quantity of fugitive gas and/or a type of fugitive gas leak, as detailed herein. In short, the method 160 can timely detect, accurately quantify, and remediate a fugitive gas leak (e.g., based on a quantity of fugitive gas and/or a type of fugitive gas leak).

Having thus described several illustrative embodiments of the present disclosure, those of skill in the art will readily appreciate that yet other embodiments may be made and used within the scope of the claims hereto attached. It will be understood, however, that this disclosure is, in many respects, only illustrative. Changes may be made in details, particularly in matters of shape, size, arrangement of parts, and exclusion and order of steps, without exceeding the scope of the disclosure. The disclosure's scope is, of course, defined in the language in which the appended claims are expressed.

**Claims**

1. A system (10, 50) for probability based fugitive gas leak detection, the system comprising:

   an equipment group (23a, 23b) including:

   a plurality of potential fugitive gas sources (24a, 24b, 24c, 24d, 24e, 24f, 24g); and
   a plurality of gas sensors (20a, 20b, 20c, 20d, 20e, 20h, 20i) positioned at respective locations in proximity to the plurality of potential fugitive gas sources, wherein each of the plurality of gas sensors is configured to detect gas concentrations over a time period; and

   a supervisor (12) communicatively coupled to the equipment group, the supervisor being configured to:

   receive time-series data indicative of the detected gas concentrations over the time period;
   determine a probability matrix based at least on coordinates of the plurality of gas sensors and coordinates of the plurality of potential fugitive gas sources; and
   identify, from the plurality of potential fugitive gas sources, a potential fugitive gas source as an actual fugitive gas source based on the probability matrix and the time-series data.

2. The system of claim 1, wherein the plurality of gas sensors are configured to detect hydrogen, hydrogen sulfide, carbon dioxide, methane, carbon monoxide, or any combination thereof.

3. The system of any one of claims 1-2, wherein the plurality of gas sensors are located external to and a distance away from the plurality of potential fugitive gas sources.

4. The system of claim 3, wherein the plurality of gas sensors are a hazardous area certified.

5. The system of claim 4, wherein the plurality of gas sensors are located a distance away from the plurality of potential fugitive gas sources.

6. The system of claim 5, wherein the distance is in a range from about 1 meter to about 20 meters.

7. The system of claim 5, wherein the distance is a fixed distance.

8. The system of any one of claims 1-7, wherein the supervisor is configured to identify the potential fugitive gas source as an actual fugitive gas source responsive to a fugitive gas value associated with the potential fugitive gas source exceeding a fugitive emission threshold, being a highest fugitive gas value, or both.

9. The system of any one of claims 1-8, wherein the detected gas concentrations are real-time gas concentrations detected over a rolling time window during on-line operation of one or more of the plurality of potential fugitive gas sources.

10. The system of claim 9, wherein the real-time gas concentrations are detected periodically.

11. The system of any one of claims 1-10, wherein the probability matrix is determined based a distance between the coordinates of the plurality of gas sensors and the coordinates of the plurality of potential fugitive gas sources.

12. The system of any one of claims 1-11, wherein the at least one potential fugitive gas source is identified based on a sum of the gas concentrations detected by at least one gas sensor of the plurality of gas sensors over the time period.

13. The system of any one of claims 1-12, wherein the supervisor is configured to filter the plurality potential fugitive gas sources prior to identifying the at least one potential fugitive gas sources as the actual fugitive gas source, wherein the supervisor is configured to filter the plurality of potential fugitive gas sources using a minimum number of events filter, a minimum duration filter, a minimum event density filter, or any combination thereof.

14. A method for probability based fugitive gas leak detection in an environment including the system of any one of claims 1-13, the method comprising:

receiving, via a network, time-series data indicative of gas concentrations of one or more gas events detected by the plurality of gas sensors during a rolling time window;

determining a probability matrix for each equipment group of the plurality of equipment groups, wherein the probability matrix is determined based at least on coordinates of the gas sensors and coordinates of the potential fugitive gas source, and wherein the probability matrix includes probability values that correspond to combinations of an individual gas sensor and an individual potential fugitive gas source and indicate a probability that a gas concentration detected by the individual gas sensor originated from the individual potential fugitive gas source;

based at least on the gas concentrations detected by the plurality of gas sensors and the probability values the probability matrix for an equipment group of the plurality of equipment group, identifying the individual potential fugitive gas source as an actual fugitive gas source;

quantifying a volume of fugitive gas emitted by the actual fugitive gas source; and

remediating the actual fugitive gas source.

15. The method of claim 14, further comprising identifying the potential fugitive gas source as the actual fugitive gas source in the absence of real-time weather data for the environment.

FIG. 1

FIG. 2

140

```
┌─────────────────────────────────────┐
│   RECEIVE TIME-SERIES DATA           │ ─── 142
└─────────────────────────────────────┘
                  │
                  ▼
┌─────────────────────────────────────┐
│   DETERMINE A PROBABILITY MATRIX     │ ─── 144
└─────────────────────────────────────┘
                  │
                  ▼
┌─────────────────────────────────────┐
│  IDENTIFY AN ACTUAL FUGITIVE GAS     │
│              SOURCE                  │ ─── 146
└─────────────────────────────────────┘
                  │
                  ▼
┌─────────────────────────────────────┐
│   QUANTIFY A VOLUME OF FUGITIVE GAS  │ ─── 148
└─────────────────────────────────────┘
```

FIG.3

160

142 RECEIVE TIME-SERIES DATA

144 DETERMINE A PROBABILITY MATRIX

146 IDENTIFY AN ACTUAL FUGITIVE GAS SOURCE

148 QUANTIFY A VOLUME OF FUGITIVE GAS

150 REMEDIATE THE ACTUAL FUGITIVE GAS SOURCEAS

FIG.4

## EUROPEAN SEARCH REPORT

**Application Number**

EP 25 17 1503

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/357232 A1 (BRANDT DUNCAN [US] ET AL) 10 November 2022 (2022-11-10) * paragraph [0003]; figures 1,5,6,7B,8-18 * * paragraph [0007] * * paragraph [0054] - paragraph [0064] * * paragraph [0120] - paragraph [0167] * ----- | 1-15 | INV. G01M3/16 G01M3/20 G06Q10/20 G01N33/00 |
| A | NAGESWARAN SIDDESH ET AL: "Source Localization and Bayesian leak magnitude inference of sparse wireless sensor data to detect fugitive methane leak", 2022 IEEE INTERNATIONAL CONFERENCE ON BIG DATA (BIG DATA), IEEE, 17 December 2022 (2022-12-17), pages 4868-4875, XP034281944, DOI: 10.1109/BIGDATA55660.2022.10020974 [retrieved on 2023-01-26] * the whole document * ----- | 1-15 | |

**TECHNICAL FIELDS SEARCHED (IPC)**

G01M
G06Q
G01N

The present search report has been drawn up for all claims

| Place of search | Date of completion of the search | Examiner |
|---|---|---|
| The Hague | 18 September 2025 | Foster, Keir |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 17 1503

This annex lists the patent family members relating to the patent documents cited in  the above-mentioned European search report.
The members are as contained in the European Patent Office EDP file on
The European Patent Office is in no way liable for these particulars which are merely given for the purpose of information.

18-09-2025

| Patent document cited in search report | Publication date | Patent family member(s) | Publication date |
|---|---|---|---|
| US 2022357232 A1 | 10-11-2022 | CA 3220278 A1 | 10-11-2022 |
| | | US 2022357230 A1 | 10-11-2022 |
| | | US 2022357232 A1 | 10-11-2022 |
| | | US 2022357234 A1 | 10-11-2022 |
| | | US 2025076267 A1 | 06-03-2025 |
| | | WO 2022235681 A1 | 10-11-2022 |

EPO FORM P0459

For more details about this annex : see Official Journal of the European Patent Office, No. 12/82